# EUROPEAN PATENT APPLICATION

(11) **EP 2 573 569 A1**
(43) Date of publication of application: **27.03.2013**
(21) Application number: 12186076.1
(22) Date of filing: 26.09.2012
(51) Int. Cl.: G01N 33/68

(54) **Antibodies, compositions, and assays for detection of cardiac disease**

(30) Priority: 26.09.2011 US 201113245398
(71) Applicant: Nour Heart, Inc., Annapolis, MD 21403 (US)
(72) Inventor: Elgebaly, Salwa A, Annapolis, MD 21403 (US)
(74) Representative: Steinecke, Peter

(57) **Abstract**

The present invention provides antibodies, devices, and immunoassays for detection of ischemic cardiac events (unstable angina and heart attack) in patients experiencing chest pain. The invention allows for rapid determination of the cause of chest pain, and allows for differentiation of chest pain due to ischemic cardiac events and other causes. The invention provides antibodies that specifically bind to the epitope f-MII and the epitope f-MLF.

SEQUENCE LISTING

<110>  ELGEBALY, Salwa A

<120>  ANTIBODIES, COMPOSITIONS, AND ASSAYS FOR DETECTION OF CARDIAC
       DISEASE

<130>  P40559

<140>  U2 13/245,398
<141>  2011-09-26

<160>  6

<170>  PatentIn version 3.4

<210>  1
<211>  11
<212>  PRT
<213>  Homo sapiens

<400>  1

Met Ile Ile Asn His Asp Asp Glu Arg Lys Cys
1               5                   10


<210>  2
<211>  11
<212>  PRT
<213>  Homo sapiens

<400>  2

Met Ile Ile Asn His Asp Asp Glu Arg Lys Cys
1               5                   10


<210>  3
<211>  11
<212>  PRT
<213>  Artificial Sequence

<220>
<223>  Screening antigen for anti-Nourin specificity

<400>  3

Met Ile Ile Asn His Glu Glu Asp Lys Arg Cys
1               5                   10


<210>  4
<211>  11
<212>  PRT
<213>  Homo sapiens

<400>  4

Met Leu Phe Gly Gly Asp Asp Glu Arg Lys Cys
1               5                   10


<210>  5
<211>  11

<212>  PRT
<213>  Homo sapiens

<400>  5

Met Leu Phe Gly Gly Asp Asp Glu Arg Lys Cys
1               5                   10


<210>  6
<211>  11
<212>  PRT
<213>  Artificial Sequence

<220>
<223>  Screening antigen for anti-nourin antibodies

<400>  6

Met Leu Phe Ala Ala Glu Glu Asp Lys Arg Cys
1               5                   10

## Description

### BACKGROUND OF THE INVENTION

### Field of the Invention

The present invention relates to the field of medicine. More specifically, the invention relates to detection of, and differentiation between, cardiac diseases or disorders using antibodies that detect polypeptides released as a result of certain cardiac events.

### Description of Related Art

Heart disease is the leading cause of death in women and men in the United States and worldwide. It is a major cause of disability, with 935,000 heart attacks yearly in the U.S. and 600,000 deaths.

Ischemia of the heart occurs as a result of diminished blood flow to heart tissue. If this reduction is brief (less than 15 minutes), the ischemic injury is reversible, as seen in unstable angina patients. But if reduced blood blow is persistent for an extended period of time more than 15 minutes, irreversible necrotic damage (cell death) occurs leading to heart attack. Currently, no blood test can specifically identify unstable angina patients. Tests for heart attacks cannot be done until several hours (four to six hours) after a patient has experienced a heart attack.

The root cause of acute coronary syndromes (ACS) is unstable plaque in the coronary artery. These syndromes represent a continuum of ischemic disease ranging from unstable angina to heart attack (acute myocardial infarction-AMI) and large areas of heart cell death. Each year between 6 to 10 million individuals come to Emergency Rooms (ER) with clinical signs and symptoms of ACS including chest pain. Of the 6-10 million patients presenting annually to the ER with chest pain, up to 90% do not have a heart cause for their symptoms. In the U.S. the high rate of chest pain admissions of non-heart origin is as high as 60%. Accordingly, there is an urgent need for a quick blood test that can accurately rule in or out the 10% heart patients, who are equally divided (50:50) between unstable angina and heart attack. Currently, there is no biomarker to identify patients with unstable angina seen in the ER with chest pain. The misdiagnosis of unstable angina patients in the ER is the highest source of medical malpractice lawsuits in the U.S.

It is known in the art that acute myocardial infarction (AMI) is associated with the release of certain small molecules and proteins as a result of damage to cardiac tissue. Currently, Troponin release is the most widely used marker for AMI. However, troponin as a marker for AMI has certain drawbacks. For example, the troponin complex is not highly stable as an extracellular protein, and thus its usefulness as a marker for AMI is diminished in samples that have been stored.

Although, Troponins are the "Gold Standard" for determining if a patient has had a heart attack, it is a marker of "cell death" and requires four to six hours of waiting after the onset of chest pain in order for the markers to appear in the blood; however, at this late stage, treatment to save ischemic heart tissues might be missed. Troponins also have low specificity where 50% of the time the elevated levels of Troponins give false positives for non-ischemic heart attack patients such as kidney renal failure and congestive heart failure. There thus exists a need in the art for a better test for unstable angina and myocardial infarction.

### SUMMARY OF THE INVENTION

The present invention provides an assay for detection of one or more small molecules that are released as a result of certain heart disorders, including AMI and unstable angina (UA). Unlike the troponin assay currently in use, the assay according to the invention uses a biomarker for "reversible cell injury" before "death". The assay can be used to identify unstable angina patients seen in the ER with chest pain, and also can be used to distinguish between patients with unstable angina, heart attack, and non-heart related symptoms of chest pain. Therefore, the assay can (1) identify unstable angina patients and reduce medical malpractice costs associated with missed heart attacks; (2) complement and enhance the usefulness of the Troponins tests to rule in or out unstable angina and heart attack; and (3) unlike Troponins, it can immediately identify heart attack patients when they arrive at the ER and eliminate the current required four to six hours of waiting and thus allows crucial therapy to save heart muscles from dying. It can also reduce unnecessary health care expenses.

The assay involves the use of at least one, and preferably two, antibodies that specifically bind to molecules that are released as a result ofUA and AMI. More specifically, the invention provides for the use of an antibody that specifically binds to the epitope f-MII (formylmethionine-isoleucine-isoleucine), for the use of an antibody that specifically binds to the epitope f-MLF (formylmethionine-leucine-phenylalanine), or both, to detect peptides or polypeptides that are released from cardiac tissue cells upon an episode of UA and AMI. The antibodies, used alone or in combination, can thus be used to detect UA and AMI and to diagnose the cause of chest pain in cardiac patients. Importantly, the antibodies can differentiate between patients experiencing or having recently experienced UA and AMI and those having chest pain, but not experiencing of having ACS.

In a first aspect, the invention provides an antibody (referred to herein at times as "Nour001-A") that specifically binds the epitope f-MII. The antibody can be raised, e.g., in mice, using conventional techniques known in the art using about 10 micrograms of antigen and about one month between injection and collection of sera. To improve yield, a booster injection can be given at about three weeks after the initial injection. The antibody thus raised is considered polyclonal. To the inventor's knowledge, this is the first disclosure of an antibody specifically raised against the epitope f-MII.

In another aspect, the invention provides an assay for determining if a molecule that binds to the Nour001-A antibody is present in a sample. In general, the assay comprises combining a sample with the Nour001-A antibody, and detecting binding of the antibody to the molecule. Specific binding of the molecule to the antibody is indicative ofUA or AMI. As such, the assay is an immunoassay for the molecule. Any immunoassay format known in the art can be used as the assay according to the invention. A preferred assay is an ELISA assay. The assay can be used to detect one or more peptides or polypeptides in a sample. In exemplary embodiments, the peptide or polypeptide is released from cardiac tissue of a subject (used interchangeably herein with "patient") who has suffered UA and/or AMI. The assay thus may be a diagnostic assay for UA and/or AMI. However, the assay also may be an assay for detection and quatification of levels of such peptides and polypeptides in cardiac tissues under normal or stressed conditions.

Additionally or alternatively, the assay can be practiced to determine if a peptide or polypeptide that binds to an antibody specifically raised against the epitope f-MLF (also referred to herein as the "Nour001-D" antibody) is present in a sample, where the peptide or polypeptide originates from a human. Prior work with antibodies specific for f-MLF has focused on its use to detect bacterial proteins in samples. However, to the inventor's knowledge, the present invention is the first recognition that such antibodies can be used to detect human peptides or polypeptides, and in particular peptides and polypeptides released from human cardiac tissue upon UA or AMI. As with the first embodiment of the immunoassay of the invention, the assay according to this embodiment comprises combining a sample with the Nour001-D antibody, and detecting binding of the antibody to a human peptide or polypeptide. Specific binding of the molecule to the antibody is indicative ofUA or AMI. As such, the assay is an immunoassay for the molecule. A preferred assay format is an ELISA assay. The assay may be practiced to achieve any of the results discussed above with regard to the Nour001-A antibody.

Because the Nour001-A and Nour001-D antibodies show a similar binding profile, they can be used in conjunction with each other to provide a primary detection assay and a confirmatory detection assay for peptides and polypeptides release from cardiac tissue as a result of AMI. More specifically, it has been found that the binding profiles for both antibodies are similar, and that the binding profiles correlate with the profile for troponin release. It is to be noted that the Nour001-A and Nour001-D antibodies do not, however, bind troponin. The antibodies, alone or in combination, thus provide an assay for detection of AMI that is as indicative of AMI as the troponin release assay, but does not rely on troponin detection. In contrast to a troponin assay, the present assay, using Nour001-A, Nour001-D, or both, can detect UA as well as AMI. Importantly, it has been determined that the peptides and polypeptides detected using the Nour001-A andNour001-D antibodies are much more stable than troponin. As such, the assays according to the present invention can advantageously be used to detect UA and AMI using relatively old samples that have been stored.

In one embodiment, the assay is a method for determining whether a sample comprises biological material of a subject that has experienced unstable angina or acute myocardial infarction, in which the method comprises combining an antibody that specifically binds to a polypeptide comprising the epitope sequence f-MLF with the sample under conditions where the antibody can specifically bind to the epitope; and detecting binding of the antibody to the polypeptide. Specific binding is indicative ofUA and/or AMI. In exemplary embodiments, the antibody is the Nour001-D antibody. The method can further comprise combining an antibody that specifically binds to a polypeptide comprising the epitope sequence f-MII with the sample under conditions where the antibody can specifically bind to the epitope; and detecting binding of the antibody to the polypeptide. Specific binding is indicative of UA or AMI. Preferably, the assay performed with the antibody that binds f-MII is performed in a separate assay chamber, on a separate solid support, or on a separate portion of a single solid support, than the initial assay. In exemplary embodiment, the second antibody is the Nour001-A antibody.

In alternative embodiments, the method can be a method of determining whether a sample comprises biological material of a subject that has experienced unstable angina or acute myocardial infarction, in which the method comprises combining an antibody that specifically binds to a polypeptide comprising the epitope sequence f-MII with the sample under conditions where the antibody can specifically bind to the epitope; and detecting binding of the antibody to the polypeptide. Specific binding is indicative ofUA or AMI. In exemplary embodiments, the antibody is the Nour001-A antibody. The method can further comprise combining an antibody that specifically binds to a polypeptide comprising the epitope sequence f-MLF with the sample under conditions where the antibody can specifically bind to the epitope; and detecting binding of the antibody to the polypeptide. Specific binding is indicative of UA or AMI. Preferably, the second assay is performed in a separate assay chamber, on a separate solid support, or on a separate portion of a single solid support, than the initial assay. In exemplary embodiments, the second antibody is the Nour001-D antibody.

The methods of the invention can be practiced on any biological sample. However, it is preferred that the sample is one originating, derived from, or otherwise containing human peptides and/or polypeptides. As such, in exemplary embodiments, the subject is a human subject and the sample is obtained from that subject. Because the antibodies are particularly well suited for detecting peptides or polypeptides released from cardiac tissue cells upon UA and AMI, in exemplary embodiments, the sample contains peptides or polypeptides originating from cardiac tissue or cells. The sample thus can contain cardiac tissue or cell lysates of cardiac tissue. Where samples contain cardiac tissue or cell lysates thereof, the assay is typically performed for research purposes to characterize the physiology of cardiac tissues and cells. Because the peptides or polypeptides are released from cardiac cells upon UA and AMI, in other exemplary embodiments, the sample is blood or a protein-containing fraction of blood.

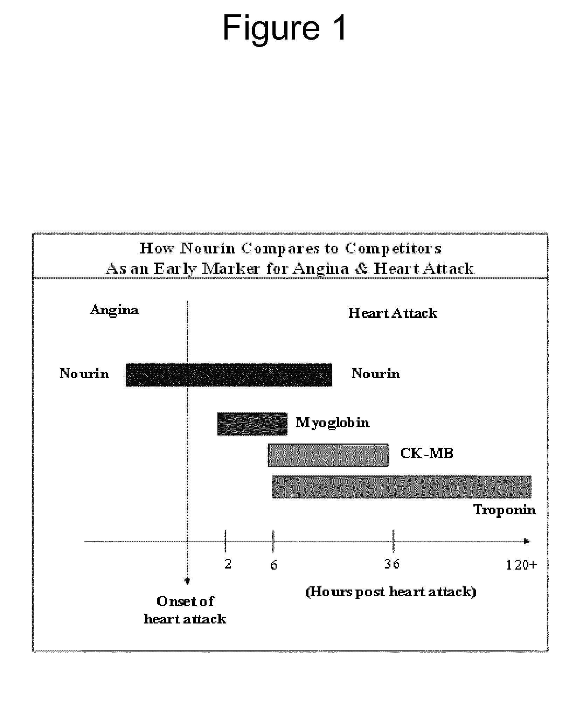

As shown in Figure 1, the assays of the invention can detect UA at least two hours prior to onset of AMI, immediately upon AMI, and up to at least 32 hours after AMI. The ability to detect UA and the ability to detect AMI immediately and within two hours after AMI makes the present assays superior to other assays for AMI known in the art. Figure 1 shows that assays that detect myoglobin, CK-MB, and troponin are not capable of detecting UA or detecting AMI within two hours of a heart attack. Furthermore, the assays of the present invention, unlike assays currently available in the market, can detect patients with UA who do not proceed to heart attack.

The invention also provides compositions comprising the antibodies of the invention. The compositions can be useful for any number of purposes, including for storage of the antibodies and for use of the antibodies in immunoassays. In general, compositions according to the invention are aqueous compositions that comprise one of the antibodies in an amount sufficient to detect the presence of a peptide or polypeptide released from cardiac cells during an episode of UA or AMI. The compositions can comprise any number of other substances, such as stabilizers for long-term storage of the antibodies, serum or other blood components, and some or all of the reagents needed to perform an immunoassay using the antibodies of the invention.

In embodiments, the composition comprises an antibody that specifically binds to the epitope f-MLF, and an aqueous sample comprising biological material of a human subject who has experienced unstable angina or acute myocardial infarction, wherein the sample comprises a peptide or polypeptide that is released from cells of the subject as a result of the unstable angina or acute myocardial infarction. Preferably, the antibody is the Nour001-D antibody. In embodiments, the antibody is bound to the polypeptide to form a complex. In some embodiments, the antibody-polypeptide complex is bound by detectable reagent, such as those commonly used in immunoassays.

In other embodiments, the composition comprises an antibody that specifically binds to the epitope f-MII, and an aqueous sample comprising biological material of a human subject who has experienced unstable angina or acute myocardial infarction, wherein the sample comprises a polypeptide that is released from cells of the subject as a result of the unstable angina or acute myocardial infarction. Preferably, the antibody is the Nour001-A antibody. As in the other embodiment, the antibody can be found in the composition bound to the polypeptide to form a complex, and the complex can be bound by detectable reagent.

In view of the usefulness of the antibodies in detecting UA and AMI, the present invention provides devices for such detection. Broadly speaking, the devices according to the invention comprise a solid support to which is bound one or both of the Nour001-A and Nour001-D antibodies. The device can take the form of any suitable immunoassay device known in the art, including, but not limited to, ELISA plates, lateral flow devices, and flow-through devices. As such, the solid support can be any suitable support known in the art for use in immunoassays, including but not limited to plastic, nylon, nitrocellulose, and the like.

In exemplary embodiments, the device is a device for detecting the presence of a peptide or polypeptide released from cells as a result of unstable angina or acute myocardial infarction, in which the device comprises a solid support to which is bound an antibody that specifically binds to the epitope f-MII. Preferably, the antibody is the Nour001-A antibody. In certain embodiments, the solid support further comprises an antibody that specifically binds to the epitope f-MLF bound to the support. Preferably, the antibody is the Nour001-D antibody.

### BRIEF DESCRIPTION OF THE DRAWINGS

The accompanying figures, which are incorporated in and constitute a part of this specification, provide data supporting the claims of the present document, and together with the written description, serve to explain certain principles of the invention.

Figure 1 indicates the timeframe under which various assays for UA and AMI are useful. Specifically, the figure indicates that the present assays can detect UA up to at least two hours prior to a heart attack, and up to at least 32 hours after a heart attack. The present assay is capable of detecting UA in patients, regardless of whether or not they ultimately suffer a heart attack. The myoglobin assay known in the art cannot detect UA and can detect AMI only between about 2 hours and 8 hours after AMI. The CK-MB assay known in the art likewise cannot detect UA, and is useful only between 6 hours and 36 hours after a heart attack. The troponin assay is likewise limited to use only after 8 hours post-heart attack, although it can be used up to 120 hours or more after a heart attack.

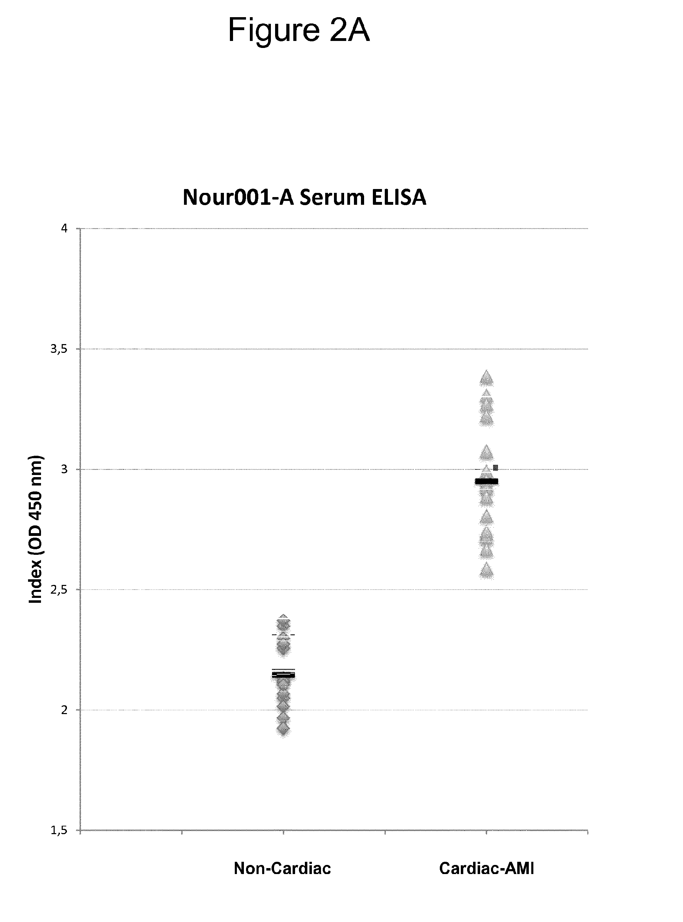

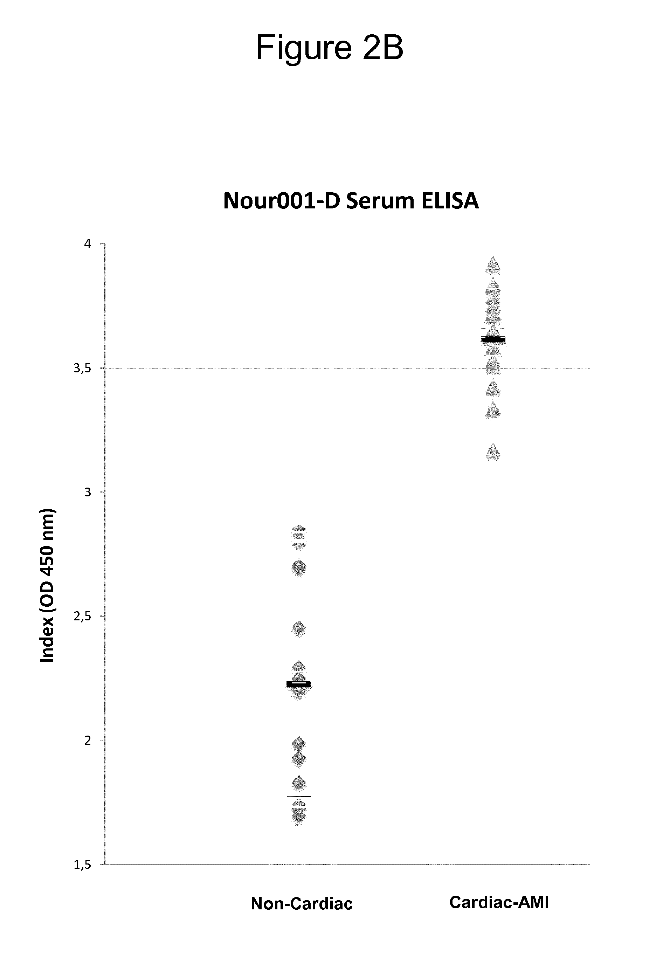

Figure 2, Panel A, shows a graph indicating that the Nour001-A antibody can be used in an ELISA format to specifically detect a protein or polypeptide in serum of a patient who has suffered AMI, and to differentiate that serum from serum taken from a patient suffering chest pains, but not AMI.

Figure 2, Panel B, shows a graph indicating that the Nour001-D antibody can be used in an ELISA format to specifically detect a protein or polypeptide in serum of a patient who has suffered AMI, and to differentiate that serum from serum taken from a patient suffering chest pains, but not AMI.

### EXAMPLES

The invention will be further explained by the following Examples, which are intended to be purely exemplary of the invention, and should not be considered as limiting the invention in any way.

Example 1: Production of the Nour001-A and Nour001-D Antibodies

A short peptide sequence derived from the N-terminus of Nourin-1 was used to generate antibodies in mice. The amino acid sequence f-MIINHDDERKC (SEQ ID NO:1) was chemically synthesized and purified using HPLC. This peptide was conjugated to KLH using a proprietary method of Precision Antibody (Columbia, Maryland), and mice were immunized. Tailbleeds were collected for determination of antibody titer at three weeks, and final cardiac bleeds were performed at four weeks to collect final sera.

The collected sera were tested for specificity of binding to the immunogen as follows. Diluted sera were combined with a control peptide (MIINHDDERKC; SEQ ID NO:2) in excess to bind and remove antibodies in the sera that bind to any portion of the immunogen other than a portion that includes the formyl-methionine. The "cleared" sera was tested against a screening antigen having the sequence f-MIINHEEDKRC (SEQ ID NO:3). As can be seen from a comparison of the sequences, the screening antigen shows identity to the immunogen only at the N-terminal five residues. Results of an ELISA with the "cleared" sera and screening antigen showed that the "cleared" sera contained antibodies that specifically bound to the formylated N-terminal sequence.

In a similar fashion, the sequence f-MLFGGDDERKC (SEQ ID NO:4) was used to generate the Nour001-D antibody. In brief, sera collected from immunized mice were tested for specificity of binding to the immunogen as follows. Diluted sera were combined with a control peptide (MLFGGDDERKC; SEQ ID NO:5) in excess to bind and remove antibodies in the sera that bind to any portion of the immunogen other than a portion that includes the formyl-methionine. The "cleared" sera was tested against a screening antigen having the sequence f-MLFAAEEDKRC (SEQ ID NO:6). As can be seen from a comparison of the sequences, the screening antigen shows identity to the immunogen only at the N-terminal three residues. Results of an ELISA with the "cleared" sera and screening antigen showed that the "cleared" sera contained antibodies that specifically bound to the formylated N-terminal sequence.

Example 2: Differentiation of AMI From Non-AMI in Chest Pain Patients Using Nour001-A Antibodies

Previous studies by the inventor had shown that a small protein (approximately 3 kDa), referred to as Nourin-1, is released shortly after an ischemic cardiac event, e.g. ischemic cardiac arrest, unstable angina, and heart attack (AMI). Those studies relied on either a neutrophil chemotaxis assay or on an immunoassay using monoclonal sera raised against the full-length Nourin-1 protein. The studies also relied on samples taken from patients known to have experienced a cardiac ischemic event. However, while the studies identified Nourin-1 as an early marker of myocardial ischemia, the studies did not address whether Nourin-1 could be used as a specific marker for ischemic events as compared to other causes of chest pain. Furthermore, the studies did not show that a particular region of Nourin-1 can serve as an antigen for raising antibodies, or that antibodies to non-Nourin-1 sequences could be used to identify cardiac ischemic events.

To determine the suitability of the Nour001-A antibody in detecting AMI, serum samples were taken from emergency room patients complaining of chest pain. The samples were collected approximately eight hours after onset of pain. The samples were tested for troponin levels to determine if the samples were from patients suffering AMI or some other cause of chest pain. The cut-off level for classifying a sample as troponin positive was a troponin level of greater than 0.07 ng/ml.

Troponin (+) and troponin (-) samples were used in a direct ELISA assay. For the Nourin direct ELISA assay, Nourin-BSA (antigen) or no antigen (buffer) was bound to the bottom of individual 96 well plates. After incubation at room temperature for one hour, the plates were washed to remove any none bound Nourin-BSA from the well. Next, either 1) buffer + polyclonal anti-Nourin antibodies (Nour001-A) or 2) patients sera samples + polyclonal anti-Nourin antibodies (Nour001-A) were added and the plate is incubated at room temperature for one hour. Next, each well was washed to remove unbound anti-Nourin antibodies. The anti-Nourin that was bound to the Nourin-BSA coupled to the bottom of the well was detected using standard anti-mouse IgG antibodies and Strepavidin-preoxidase reagents and read at OD 450 nm.

For the f-MLF direct ELISA assay, f-MLF-BSA (antigen) or no antigen (buffer) was bound to the bottom of individual 96 well plates. After incubation at room temperature for one hour, the plates were washed to remove any none bound f-MLF-BSA from the well. Next, either 1) buffer + polyclonal anti-F-MLF antibodies (Nour001-D) or 2) patients sera samples + polyclonal anti-F-MLF antibodies (Nour001-D) were added and the plate was incubated at room temperature for one hour. Next, each well was washed to remove unbound anti-F-MLF antibodies. The anti-F-MLF that was bound to the f-MLF-BSA coupled to the bottom of the well was detected using standard anti-mouse IgG antibodies and Strepavidin-preoxidase reagents and read at OD 450 nm.

The data collected is presented in Figure 2, Panel A. As shown in the figure, the troponin (-) samples (labeled "Non-Cardiac") showed an average OD reading of approximately 2.2, whereas the troponin (+) samples (labeled "Cardiac-AMI") showed an average OD reading of approximately 2.9, with no overlap between individual samples of the two types. The Nour001-A antibody binding profile thus correlates well with the troponin release profile. As such, the Nour001-A antibody is well suited as a detection reagent for AMI.

Presumably, the Nour001-A antibody detects Nourin-1; however, this presumption has not been verified. As such, it is possible that the Nour001-A antibody binds one or more other peptide or polypeptide. Regardless of the identity of the molecule(s) bound by the antibody, the data presented herein show that the Nour001-A antibody can be used not only to identify a peptide or polypeptide released upon AMI, but also that the antibody can differentiate between patients suffering AMI and patients complaining of chest pain, but not suffering AMI. The Nour001-A antibody thus can be used in diagnostic assays to diagnose AMI.

Example 3: Differentiation of AMI From Non-AMI in Chest Pain Patients Using Nour001-D Antibodies

Antibodies specific for the epitope f-MLF are known in the art and have been previously used for detection of bacterial proteins having this sequence. However, to the inventor's knowledge, to date there have been no studies performed and published indicating that such antibodies can be used to detect a human protein, or to indicate that such proteins have any suitability in diagnostic assays based on human proteins.

To determine the suitability of the Nour001-D antibody in detecting AMI, serum samples were taken from emergency room patients complaining of chest pain. The samples were collected approximately eight hours after onset of pain. The samples were tested for troponin levels to determine if the samples were from patients suffering AMI or some other cause of chest pain. The cut-off level for classifying a sample as troponin positive was a troponin level of greater than 0.07 ng/ml.

Troponin (+) and troponin (-) samples were used in a direct ELISA assay in accordance with the description above. The data collected is presented in Figure 2, Panel B. As shown in the figure, the troponin (-) samples (labeled "Non-Cardiac") showed an average OD reading of approximately 2.25, whereas the troponin (+) samples (labeled "Cardiac-AMI") showed an average OD reading of approximately 3.6, with no overlap between individual samples of the two types. The Nour001-D antibody binding profile thus correlates well with the troponin release profile. As such, the Nour001-D antibody is well suited as a detection reagent for AMI.

Presumably, the Nour001-D antibody detects Nourin-1; however, this presumption has not been verified. As such, it is possible that the Nour001-D antibody binds one or more other peptide or polypeptide. Regardless of the identity of the molecule(s) bound by the antibody, the data presented herein show that the Nour001-D antibody can be used not only to identify a peptide or polypeptide released upon AMI, but also that the antibody can differentiate between patients suffering AMI and patients complaining of chest pain, but not suffering AMI. The Nour001-D antibody thus can be used in diagnostic assays to diagnose AMI.

Example 4: Confirmation of Prior Results on Stored Samples

To determine whether the Nour001-A antibody could reproducibly detect AMI samples from non-AMI samples, the samples used in Example 2 were re-tested. Specifically, the samples used in Example 2 were stored for one month at -20°C, then thawed and subjected to the same ELISA test procedure as described in Example 2. The data obtained was similar to the earlier results, showing a difference between troponin (+) samples and troponin (-) samples. In this repeat study, the troponin (+) samples showed an average OD of approximately 2.4, whereas the troponin (-) samples showed an average OD of approximately 1.8. These results show not only that the results obtained using the antibodies of the invention are reproducible, but also that the antibodies can be used to detect peptides and polypeptides after a relatively long period of storage. Lack of stability of troponin is a significant drawback to its use as a marker for AMI, a drawback that is overcome by the present invention.

Example 5: Differentiation of AMI and Unstable Angina (UA) Samples From Samples Taken From Patients With Chest Pain But No AMI or UA

To further characterize the Nour001-A antibody for its usefulness in identifying samples taken from patients suffering a cardiac ischemic event, or for diagnosing AMI or UA, samples were obtained from emergency room patients and tested in a blind study to determine if the Nour001-A antibody could differentiate between AMI or UA samples and samples taken from patients experiencing chest pain, but not suffering AMI or UA. The samples were initially obtained from patients within eight hours of onset of chest pain, then stored at -70°C for three years. The samples were processed as discussed above, with the exception that samples were coded rather than labeled to ensure that there would be no bias introduced during processing of the samples.

The assay showed a statistically significant difference (p=0.012 using the Kruskal-Wallis test) between samples from patients with AMI, unstable angina, and other non-cardiac patients with chest pain. The assay can, therefore, identify unstable angina patients, and can distinguish them from the other AMI patients. In other words, the assay can identify UA patients with low Troponin (0.3- 0.07 ng/ml) below the heart attack cut off of 0.07 ng/ml. Additionally, the assay can significantly distinguish between cardiac patients (UA and AMI) from patients with chest pain unrelated to the heart.

Importantly to note that the assay distinguished cardiac patients from non-cardiac patients using fresh samples or samples kept frozen for three years.

The assay identifies unstable angina patients and complements and enhances the usefulness of Troponin tests to rule in or out unstable angina and heart attack. If the assay of the invention does not detect a peptide or polypeptide, then ACS can be ruled out and the patient can be released from the ER or a workup can begin to elucidate the true cause of the patient's symptoms. On the other hand, if the assay of the invention detects a peptide or polypeptide, the patient can receive therapies known to benefit ACS patients in an earlier timeframe than is possible with current assays. In addition, unlike Troponins, the assay can immediately identify heart attack patients when they arrive at the ER and eliminate the current required the long wait (4-6 hours) for Troponins results. Earlier identification of heart patients allows for early intervention to avoid permanent damage and heart attack that can lead to heart failure and death. In general, 40% to 50% of heart attack patients will suffer from heart failure.

It will be apparent to those skilled in the art that various modifications and variations can be made in the practice of the present invention without departing from the scope or spirit of the invention. Other embodiments of the invention will be apparent to those skilled in the art from consideration of the specification and practice of the invention. It is intended that the specification and examples be considered as exemplary only, with a true scope and spirit of the invention being indicated by the following claims.

## Claims

1. A method of determining whether a sample comprises biological material of a subject that has experienced an acute coronary syndrome selected from unstable angina and acute myocardial infarction, said method comprising:
combining an antibody that specifically binds to a polypeptide comprising the epitope sequence formylmethionine-isoleucine-isoleucine (f-MII), an antibody that specifically binds to a polypeptide comprising the epitope sequence formylmethionine-leucine-phenylalanine (f-MLF), or both antibodies, with the sample under conditions where the antibody can specifically bind to the epitope; and
detecting binding of the antibody to the polypeptide.

2. The method of claim 1, wherein the subject is a human subject.

3. The method of claim 1 or 2, wherein the sample is blood or a protein-containing fraction thereof.

4. The method of any one of claims 1 to 3, wherein the sample is cardiac tissue or comprises cell lysate thereof.

5. A composition comprising:
an antibody that specifically binds to the epitope f-MLF; and
an aqueous sample comprising biological material of a human subject who has experienced an acute coronary syndrome selected from unstable angina and acute myocardial infarction,
wherein the sample comprises a polypeptide that is released from cells of the subject as a result of unstable angina or acute myocardial infarction.

6. The composition of claim 5, wherein the antibody is bound to the polypeptide to form a complex.

7. The composition of claim 6, wherein the antibody-polypeptide complex is bound by detectable reagent.

8. A composition comprising:
an antibody that specifically binds to the epitope f-MII; and
an aqueous sample comprising biological material of a human subject who has experienced unstable angina or acute myocardial infarction, wherein the sample comprises a polypeptide that is released from cells of the subject as a result of the unstable angina or acute myocardial infarction.

9. The composition of claim 8, wherein the antibody is bound to the polypeptide to form a complex.

10. The composition of claim 9, wherein the antibody-polypeptide complex is bound by detectable reagent.

11. A device for detecting the presence of a polypeptide released from cells as a result of unstable angina or acute myocardial infarction, said device comprising a solid support to which is bound an antibody that specifically binds to the epitope f-MII.

12. The device of claim 11, wherein the solid support further comprises an antibody that specifically binds to the epitope f-MLF bound to the support.

13. The device of claim 11 or 12, wherein the solid support comprises plastic, nylon, nitrocellulose, or another substance suitable for use as a solid support in immunoassays.

14. A method for predicting the likelihood of a patient who is suffering from a cardiovascular disease will suffer an acute myocardial ischemic event, said method comprising:
obtaining a biological sample of cardiovascular tissue from the patient, and
detecting the presence and level ofNourin in the sample,
wherein the amount of Nourin in the sample above a threshold amount indicates the likelihood that the patient will suffer an acute myocardial ischemic event.

15. The method of claim 14, wherein the cardiovascular disease is coronary artery disease.
